# EUROPEAN PATENT SPECIFICATION

(11) **EP 3 171 821 B1**
(45) Date of publication and mention of the grant of the patent: **18.03.2020**
(21) Application number: 15824633.0
(22) Date of filing: 24.07.2015
(51) Int. Cl.: A61F 2/16

(54) **HAPTIC DEVICES FOR INTRAOCULAR LENS**
HAPTISCHE VORRICHTUNGEN FÜR INTRAOKULARLINSE
DISPOSITIFS HAPTIQUES POUR LENTILLE INTRAOCULAIRE

(30) Priority: 24.07.2014 US 201462028375 P
(43) Date of publication of application: 31.05.2017
(73) Proprietor: Anew IOL Technologies, Inc., Bristol TN 37620 (US)
(72) Inventor: HAYES, Anna S., Blue Hill, ME 04614-1043 (US)
(74) Representative: Bond, Christopher William
(86) International application number: PCT/US2015/041987
(87) International publication number: WO 2016/014940

(56) References cited:
- EP-A2- 2 111 822
- FR-A1- 2 997 624
- US-A1- 2003 135 272
- US-A1- 2010 152 846
- US-A1- 2012 330 415

## Description

### Background

### 1. Field of the Invention

This invention is directed to haptic devices for intraocular lenses that provide increased comfort and performance to a patient. In particular, the invention is directed to haptic devices and designs, that consider a two-ring configuration within which the lens optic is held, that enhance post-surgical ocular health by maintaining separation of the anterior and posterior capsule of the human eye and therefore permit circulation of the aqueous humor within the capsule. Specifically, the invention, along with its various iterations, is designed to provide refractive stability by mitigating the extent of capsular fibrosis, and, in certain instances, mitigate the onset of other post-surgical conditions, specifically Posterior Capsular Opacification, as well as posterior segment conditions such as Age Related Macular Degeneration and Retinal Detachment..

### 2. Description of the Background

An intraocular lens (IOL) is an implanted lens in the eye, usually replacing the existing crystalline lens because it has been clouded over by a cataract, or as a form of refractive surgery to change the eye's optical power. The whole device usually comprises a small plastic lens with plastic side struts, called haptics, to hold the lens in place within the capsular bag inside the eye. Haptics also form the means of attachment of lenses to other areas of the eye, including the anterior angle or sulcus, the iris, and the posterior chamber ciliary sulcus. IOLs were traditionally made of an inflexible material (e.g. PMMA) though this largely been superseded by the use of flexible materials. Most IOLs fitted today are fixed monofocal lenses matched to distance vision. However, other types are available, such as multifocal IOLs which provide the patient with multiple-focused vision at far and reading distance, toric IOLs to correct for astigmatisms, and adaptive IOLs which provide the patient with limited visual accommodation.

Intraocular lenses have been used since 1999 for correcting larger errors in myopic (near-sighted), hyperopic (far-sighted), and astigmatic eyes. This type of IOL is also called PIOL (phakic intraocular lens), and the crystalline lens is not removed. More commonly, aphakic IOLs (that is, not PIOLs) are now used for visual correction errors (especially substantial hyperopia), and implanted via Clear Lens Extraction and Replacement (CLEAR) surgery. During CLEAR, the crystalline lens is extracted and an IOL replaces it in a process that is very similar to cataract surgery: both involve lens replacement, local anesthesia, both last approximately 30 minutes, and both require making a small incision in the eye for lens insertion. Patients recover from CLEAR surgery 1-7 days after the operation. During this time, patients should avoid strenuous exercise or any activity that significantly raises blood pressure. Patients should also visit their ophthalmologists regularly for several months so as to monitor the IOL implants. CLEAR has a 90% success rate (risks include wound leakage, infection, inflammation, and astigmatism). CLEAR can only be performed on patients ages 40 and older. This is to ensure that eye growth, which disrupts IOL lenses, will not occur post-surgery.

Once implanted, IOL lenses have three major benefits. First, they are an alternative to LASIK, a form of eye surgery that may not work for people with serious vision problems. Second, effective IOL implants may eliminate the need for glasses or contact lenses post-surgery. Third, the though the cataract may return, in the form of anterior or posterior capsule opactification (which results from the proliferation of lens corticular material between the capsule and the replacement lens, this may be controlled by additional surgical procedures such as an Nd-YAG laser capsulotomy. The disadvantage is that the eye's ability to change focus (accommodate) may have been reduced or eliminated, depending on the kind of lens implanted.

While significant advances have been made in the optical quality of aphakic lenses, most lenses currently made have an overall optical thickness of one millimeter or greater at the center optical focal point (e.g. see U.S. Patent No. 4,363,142). In the late 1990's, two patents were applied for and subsequently issued for lens optics significantly thinner than the afore-referenced lens patents (U.S. Pat. Nos. 6,096,077 and 6,224,628). Although improved, the extreme thinness of the lens manufactured in accordance with 6,096,077 caused some minor distortions of the optic once in the eye, while the lens manufactured in accordance with 6,224,628 was poured of molded silicone and did not provide the desired visual acuity.

Generally, the natural lens separates the aqueous humor from the vitreous body. The iris separates the region between the cornea or anterior of the eye and the lens into an anterior chamber and a posterior chamber. The lens itself is contained in a membrane known as the capsule or capsular sac. When the lens is removed from the eye, the capsule may also be removed (capsular extraction), or the anterior portion of the capsule may be removed with the lens leaving the posterior portion of the capsule intact (extracapsular extraction). In an intraocular implant, the artificial or prosthetic lens may be inserted in the anterior chamber, the posterior chamber, or the capsular sac. The artificial lenses are usually fixedly attached within the eye, either by stitching to the iris, or by some supporting means or arms attached to the lens; in all cases the fixation mechanisms are categorized as haptics.

Several intraocular lenses designed for implant in the anterior chamber feature haptics with feet which support the lens in order to avoid the need for clips or sutures to secure the lens to the iris. The lenses work; however, sizing the lens to fit the eye is critical to avoid complications. These lenses have been made in lengths from 11.5 mm to 14 mm in 0.5 mm increments, and the thickness of the feet was about 250 microns.

A variety of lenses has been developed that provides up to four point support for the lens. The support structures for these haptics are often linked to the lens body so that the support structure should not deflect freely of the lens body, and therefore be liable to irritate portions of the eye in contact with the support structure. A variety of shapes and geometries for the lens supporting elements, or haptics, has been disclosed and described (U.S. Pat. No. 4,254,510; U.S. Pat. No. 4,363,143; U.S. Pat. No. 4,480,340; U.S. Pat. No. 4,504,981; U.S. Pat. No. 4,536,895; U.S. Pat. No. 4,575,374; U.S. Pat. No. 4,581,033; U.S. Pat. No. 4,629,460; U.S. Pat. No. 4,676,792; U.S. Pat. No. 4,701,181; U.S. Pat. No. 4,778,464; U.S. Pat. No. 4,787,902; U.S. Pat. No. Re. 33,039; U.S. Pat. No. 4,872,876; U.S. Pat. No. 5,047,052; U.K. Patent No. 2,165, 456; FR2997624; EP2111822).

Despite the advances, there remain problems with intraocular implants. For example, when an intraocular lens is inserted in the eye, an incision is made in the cornea or sclera. The incision may cause the cornea to vary in thickness, leading to an uneven surface which can cause astigmatism. The insertion of a rigid lens through the incision, even with compressible haptics, requires an incision large enough to accommodate the rigid lens (typically at least 6 mm), and carries with it the increased risk of complications, such as infection, laceration of the ocular tissues, and retinal detachment. Deformable intraocular lenses made from polymethylmethacrylate (e.g. "PMMA"), polysulfone, silicone or hydrogel may be inserted through a smaller incision. Current science and advances in surgical techniques enables incisions of less than 2 mm (micro-incision) which may prove eventually to be beneficial to the patient, though any incision of less than 3.5 mm does not require sutures.

It is preferred that the intraocular lens be capable of insertion through a small incision . U.S. Pat. No. 4,451,938 shows an intraocular lens in which the lens body is made in two pieces so that each piece may be inserted through the incision separately and then joined by dowels after insertion in the eye. U.S. Pat. No. 4,769,035 discloses a foldable lens which may be inserted through an incision about 3.5 mm in length.

When the intraocular lens is inserted in the anterior chamber of the eye, the feet of the haptics, or lens supporting elements, generally lodge in the scleral sulcus, a depression anterior to the scleral spur where the iris and the ciliary muscle join the sclera in the angle of the anterior chamber. The scleral sulcus is crossed by trabecular tissue in which are located the spaces of Fontana. The anterior chamber of the eye is filled with the aqueous humor, a fluid secreted by the ciliary process, passing from the posterior chamber to the anterior chamber through the pupil, and from the angle of the anterior chamber it passes into the spaces of Fontana to the pectinate villi through which it is filtered into the venous canal of Schlemm. The lens should be positioned so the flow of fluid through the trabecular tissue is not blocked or glaucoma may result.

Since the feet of the haptics of anterior chamber lenses rest in the scleral sulcus, the flow of fluid is blocked where the feet are in contact with the trabecular tissue. It is therefore desirable to decrease the amount of surface area of the haptic foot in contact with the trabecular tissue. At the same time, the haptic feet have sufficient height to prevent adhesive tissue or synechia from growing around the feet and anchoring them to the iris or cornea. The opening of the trabecula is about 200 microns, and the haptic feet of conventional intraocular lenses are usually on the order of 175 to 200 microns, effectively blocking the openings in the trabecula wherever the feet are in contact with the tissue.

Other lenses that are situated in the posterior chamber may attach to the ciliary sulcus or be positioned in the equator of the capsular sac. In haptics with attachment to the ciliary sulcus, appropriate dimensioning is essential to ensure proper anchoring. In haptics with attachment to the capsular equator, recent science demonstrates the need for appropriate dimensioning also, as the haptic must place the lens properly in the capsule. If the haptic is too short for the capsule, the lens can dislodge or rotate in the eye, events that can require additional surgery to correct and can also cause intraocular trauma. Additionally, haptics that are too short for the capsule do not allow the lens to provide the patient with any desired or designed focal flexibility (that is, accommodation). If the haptic is too long for the capsule, the lens can angle either posteriorly or anteriorly at a greater angle than designed, in the former case significantly reducing visual acuity at distance and risking reverse accommodation, in the latter case putting pressure on the iris and diminishing focal flexibility.

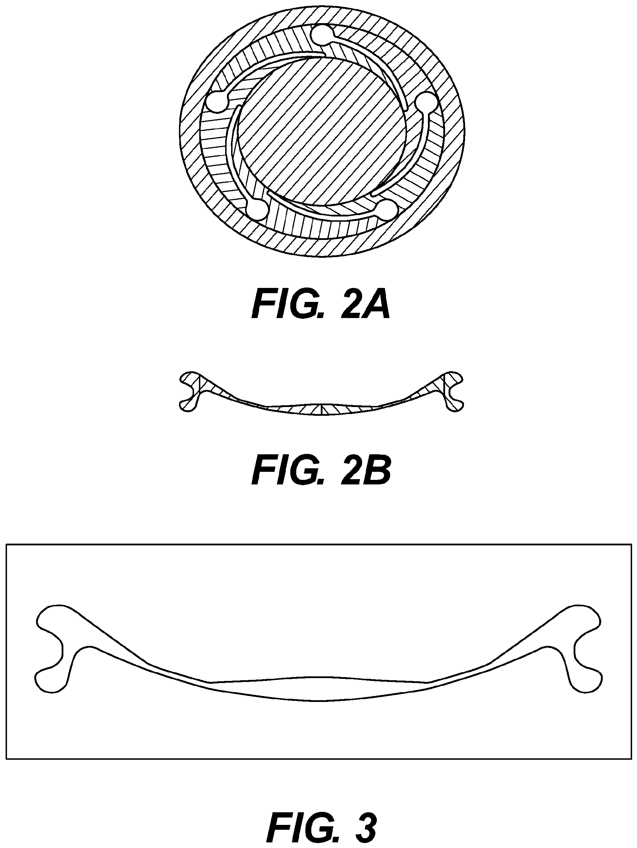

U.S. Pat. Nos. 5,258,025 and 5,480,428 describe a lens surrounded by a sheet-like "positioner" having projections called "supporting elements either at the four corners of or continuously around the positioner, the supporting elements being 0.3 mm long and 0.01 to 0.05 mm thick (7"a and 7"b of FIG. 3 of the '025 patent, 18 of the '428 patent). However, the lens is for implantation in the posterior chamber, the lens of the '428 actually having a length short enough to "float." In addition, the sheet-like nature of the positioner prevents independent deflection of the feet in response to forces applied by the eye.

In addition, the lens may place a greater or lesser degree of force on the haptic feet as the lens is compressed, depending upon construction of the lens. Since the amount of pressure for a given surface area is proportional to the force, it is desirable to decrease or distribute the amount of force placed on the haptic feet in order to diminish the force applied by the feet on the trabecular tissue. This goal is achieved by mounting the haptic arms on the ends of a flexible support bar in cantilever fashion, the support bar being offset from the lens body by a stem.

The act of surgically removing the natural lens and replacing it with an intraocular lens of whatever design gives rise to certain other possible conditions that can have a profound impact on the patient's ability to see clearly over a protracted period of time, the extent of focal accommodation that can be provided to the patient, and the effective positioning of the replacement lens in the eye. These conditions normally occur in a majority of cases but may be able to be mitigated with inventive lens and haptic designs. In particular, ophthalmologists have observed that the lens capsule will tend to atrophy over time. This is in part attributable to the fact that the replacement lens rarely occupies the entire lens capsule, and most lenses tend to flatten out the capsule, thus allowing the anterior and posterior surfaces of the capsule to adhere together, causing capsular fibrosis, atrophy, hardening, and adhesions. All these will necessarily diminish the effectiveness of any lens claiming to offer focal accommodation. In addition, it is possible that such fibrosis will cause the intraocular lens to re-position, either anteriorly or posteriorly, tilt, or decenter, any of which could cause significant change in the patient's refractive correction and quality of vision. It is possible that increased circulation of the aqueous humor can preserve the suppleness of the natural lens capsule, and preventing contact between the capsular surfaces should prevent capsular fibrosis, thereby protecting the integrity of the patient's refractive correction and overall visual acuity.

Some physicians have advocated the use of capsular tension rings to prevent capsular atrophy. However, these rings, which are situated in the lens equator , do not allow the ciliary body to influence the dimensions of the lens so as to provide for focal accommodation. Thus, whereas capsular retention rings may be effective when used in conjunction with non-accommodating or multifocal lenses, their value with premium lenses that claim accommodation is questionable.

In some cases post surgical adhesions can occur between the lens capsule and the haptic of the intraocular replacement lens. If significant enough, these adhesions can diminish the focal accommodative functions of the lens.

Posterior Capsule Opacification (PCO) is a condition that occurs in approximately 50% of cataract patients within three years after surgery. PCO is caused by the natural migration of lens epithelial cells from the anterior lens capsule to the equator Once the epithelial cells reach the equator, the cells die off leaving proteins of lens corticular material that accumulate on the posterior capsular surface in the form of Elschnig's pearls or of Soemmering's Rings. These fibroblasts that adhere to the capsule can cause significant shrinkage, and clouding of the lens. If the PCO migrates to the optical area of the capsule, vision is significantly impaired. The occurrence of PCO can be mitigated surgically by means of Nd-YAG-Laser correction, which perforates the posterior capsule with a hole that opens the optical zone of the posterior capsule. However, Nd-YAG laser capsulotomy surgery also carries risks of post-surgical complications including possible prolapse of the vitreous into the capsule (which can precipitate retinal detachment), and, as such, should be avoided if possible.

In the case of the inventive haptic designs incorporated herein, the inventors believe that the onset of PCO may be delayed or eliminated altogether through the use of appropriate haptic design to deter epithelial cell migration. In particular, 1) a haptic design that keeps the capsule open and prevents contact between the anterior and posterior surfaces may assist in mitigating PCO onset by maintaining hydration of the capsule, 2) the quality of the cataract or CLEAR surgery can assist in retarding PCO through assiduous cleaning and polishing of the anterior capsule, 3) the positioning of certain retention rings against the anterior of the capsule may arrest the migration of epithelial cells which cannot release their proteins until and unless they reach the fornix (equator) of the capsule; research demonstrates that if lens epithelial cells are dislodged from the anterior capsule they do not reattach, and 5) the positioning of certain retention rings in the fornix or against the posterior capsule may act to retain any corticular protein blasts at the periphery of the posterior capsule, and thereby prevent their aggregation in the posterior capsular optic zone. In some cases, IOL designers have found some success at mitigating the onset of PCO by configuring the posterior surface of the lens so as to provide a right angle at the junction of the lens with the posterior capsule. This configuration may be particularly applicable for those lenses that rest entirely against the posterior capsule and do not accommodate. In other cases, IOL designers have determined that the surface quality of the haptic may have some influence on PCO mitigation, though this topic continues to be debated. It is generally observed that absence of capsular fibrosis may also contribute to post-surgical capsular health and patient well-being.

### Summary of the Invention

The present invention overcomes the problems and disadvantages associated with current strategies and designs and provides new haptic devices and methods for positioning an intraocular lens in the eye, as well as designs for specific functionality to provide optimal focal flexibility and mitigate common post surgical problems.

An embodiment of the invention is directed to haptic devices that are comprised of two parallel rings connected by a pillar or several pillars of haptic material, between which an optic is suspended, such that the anterior ring makes contact with the anterior capsule at some distance from the lens equator, and the posterior ring makes contact with the posterior capsule at some distance from the lens equator, the rings connected to each other and to the framework supporting the lens optic by means of ribbons and struts that maintain suitable spacing between the rings and provide for proper positioning of the lens within the capsule. The functionality of the anterior ring is to arrest epithelial cell migration across the anterior capsule, thus preventing these cells from maturing and arriving at the capsular equator. Another functionality of the inventive anterior ring is to respond to the changes of the ciliary body in such a manner as to enable the forward motion of the lens optic within the capsule to accommodate for near vision. The functionality of the posterior ring is to protect the posterior optic zone from PCO by maintaining a suitable barrier between any pearls or fibroblasts that may develop over time and block their incursion into the area behind the lens optic. Another functionality of the posterior ring is to capture the physical forces of the ciliary body and work in conjunction with the anterior ring, the struts and the ribbons of the haptic to allow the lens optic to move within the capsule to adjust to the various stages of focal accommodation. Another functionality of the posterior ring, together with the anterior ring, the struts and ribbons is to maximize the natural circulation of the aqueous humor so as to preserve hydration throughout the lens capsule and the aqueous humor. This hydration may have the additional desirable effect of providing a mechanism whereby the spent and arrested epithelial cells can be flushed away by the aqueous humor and disposed of through the trabecular meshwork.

Another embodiment of the inventive haptic is a solid circle haptic into which are cut arced channels, preferably five, that extend from the anterior ring to the edge of the optic. These channels allow the optic to move in accommodation without distortion or decentralization, while the anterior and posterior haptic rings fix the lens centered in the capsule and maintain the capsule open.

Another embodiment of the inventive haptic is to provide for a series of easements in the struts connecting the anterior and posterior haptic rings whereby the level of force exercised on the lens is commensurate with the desired degree of accommodative movement of the lens within the eye.

Other embodiments and advantages of the invention are set forth in part in the description, which follows, and in part, may be obvious from this description, or may be learned from the practice of the invention.

### Description of the Drawings

Figures 1A-B illustrate an open loop haptic design (kidney haptic) with full anterior and posterior rings.
Figures 2A-B illustrate a full circle haptic with arced grooves.
Figure 3 illustrates another embodiment of the invention showing a lens design in cross section.
Figure 4 illustrates another embodiment of the invention showing a lens design in cross section.
Figure 5 illustrates another embodiment of the invention showing a top view of a lens design.
Figure 6 illustrates the cutaway of Figure 5.
Figure 7 illustrates a lens with spiral anterior and posterior haptics.
Figure 8 illustrates a lens with straight anterior and posterior haptics.
Figure 9 illustrates a posterior pillar system designed to bend if necessary to adjust to different capsular dimensions.
Figure 10 illustrates an embodiment of the invention showing a lens in cross section
wherein the uppermost haptic is comprised of the anterior ring designed to come into contact with the anterior capsule surface, thereby arresting lens epithelial cells' migration along the anterior capsule to the fornix.
Figures 11A-B illustrate an embodiment of the invention having one orientation tab and containing the presence of a small fin on the inner anterior surface of the anterior ring designed to create a sharper edge for arrest of migrating lens epithelial cells..
Figures 12A-B illustrate another embodiment of the invention having an orientation tab.
Figures 13A-B illustrate another embodiment of the invention.

### Description of the Invention

The haptic device is used to affix an intraocular lens within the lens capsule once the natural crystalline lens has been removed surgically. The three specific design purposes of the haptic are: i) to permit the lens to be implanted in the eye by means of an injector through an incision of less than about 3 mm; ii) to allow the lens to move within the posterior chamber of the eye in order to provide focal flexibility to the patient; and iii) to affix the lens in the lens capsule in such a way as to minimize the risk of Posterior Capsule Opacification ("PCO"), a negative consequence of lens replacement procedures that currently occurs in approximately 50% of patients within 2 to 3 years after surgery, iv) to maximize circulation of the aqueous humor within the lens capsule to minimize capsular fibrosis, and v) to provide a safe and comfortable framework for lenses with different styles of optics, with the objective of preserving as much as possible the natural physiognomy of the eye. Although intraocular lenses have been successfully implanted for several decades now, many of the haptic designs do not produce the desired results of mitigating PCO, reducing capsular fibrosis, presering the integrity of the vitreous humor and posterior segment, and/or facilitating focal flexibility (or the ability of the patient to adjust far to near vision and minimize the need for reading glasses).

A haptic device design has been surprisingly discovered that that ameliorates PCO and significantly deters capsular fibrosis. In one embodiment, the haptic of the invention consists of a pair of rings connected to each other by pillars of haptic material, and to the optic by struts or bands of the same material as the attached lens, which preferably may be any of polymethylmethacrylate, hydrophobic or hydrophilic acrylate, silicone, or blends of these materials (or of the same material as the lens).

In this embodiment, a haptic design has been surprisingly discovered that has anterior and posterior haptic feet that comprise entire rings that rest on the anterior and posterior capsules, respectively, maintaining the entire capsule open and creating a barrier at both the anterior and the posterior capsular surfaces to prevent migration of epithelial cells. In this embodiment, the haptic feet are connected by a series of struts that have open spaces between, preserving the designed distance between the rings and providing for optimal fluid circulation around the inventive lens. In this embodiment also, the anterior and posterior rings may be configured so as to arrest epithelial cell migration across the anterior capsule and incursion of PCO into the optical zone of the posterior capsule, thereby providing the potential for the patient to use the intraocular lens for a substantial period of time without adverse consequences. In this embodiment, easements may be made in the struts to accommodate smaller than normal capsules, thus providing for stable concentration of the lens optic notwithstanding potential capsular size differences or changes over time. In this embodiment additionally certain easements may be made in the inner surface of the anterior and posterior rings so as to provide for responsiveness of the lens haptic to the muscular prompts of the ciliary body.

In these embodiments, the entire dimension of the lens, including both haptics and the optic, preferably varies depending upon the measurement of the natural lens capsule. The haptic has varying points of individual tailoring, including the length of the ribbon haptic (2) and (3), and the dimension of the solid end portion of the haptic. Additionally, the haptic may be used for veterinary purposes, and its overall dimensions may be increased or reduced to fit in the lens capsule of various animals.

In the embodiments disclosed herein, rings may also be affixed to the anterior and or posterior joints or legs of such angled segments to rest in the capsule at some distance from the equator, or with one ring in the equator and the other at some distance, to mitigate the migration of epithelial cells. In such cases the rings may contain right angles at the areas of contact with the anterior or posterior surface of the capsule. The function of such rings in conjunction with the angled segments may also be to maintain the aperture of the lens capsule distant from the equator so as to provide for continuous irrigation of the region by the normal circulation mechanisms of the aqueous humor. This may preserve the natural consistency and elasticity of the lens capsule, thus ensuring prolonged functionality of the inventive lens haptic.

One function of the anterior retention ring is to arrest epithelial cells that are migrating along the anterior capsule. When these epithelial cells are removed from the capsular wall they lose their ability to adhere to any surface (i.e. once detached are not reattachable). This means that with a barrier along the anterior capsule the number of epithelial cells that arrive at the equator can be limited whence they release cortical material that can cause PCO. The posterior ring limits the extent of PCO incursion, whereas the anterior ring limits PCO creation.

Preferred materials for the intraocular lens comprises hydrophilic acrylic, hydrophobic acrylic, silicone or other suitable, and preferably a flexible material that is approved for intraocular use. Preferred materials retain sufficient molecular memory to provide for constant positioning of the lens against the inner capsular wall. It is also preferred that the acrylic material be flexible enough to change shape easily and respond to the prompts of the ciliary body, but resilient enough to resist cracking or other deterioration for decades. Contact and continued contact of the haptic rings with the lens capsule strongly hinders and even prevents migration of epithelial cells along the anterior capsule to the equator, which is the cause of Posterior Capsular Opacification (or PCO) in many post-cataract surgery patients. The preferred design also maintains the open lens capsule, thus preventing the possibility of adhesions between the anterior and posterior surfaces of the capsule. Further, the open capsule also allows the aqueous humor to circulate within the capsule, which provides for enhanced hydration of the lens capsule. This enhanced hydration provides a significant advantage over models of intraocular lenses that are primarily two-dimensional in their configuration and which stretch the lens capsule out horizontally.

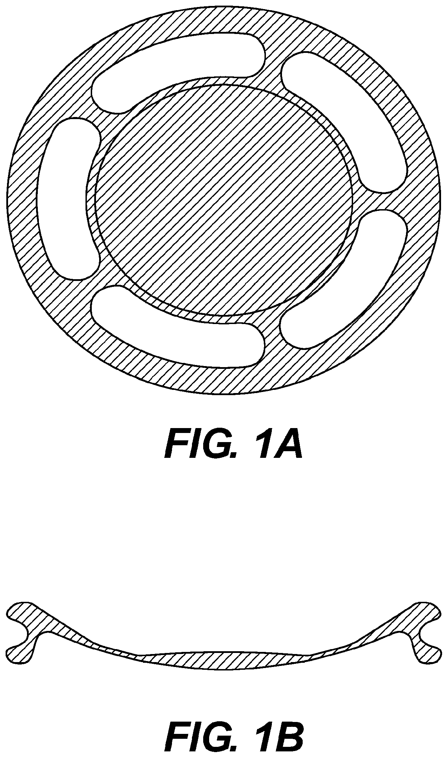

Figures 1A-B depict both top and sagittal views of the full circular haptic with ribbons and struts to create oval openings between the optic and the haptic rings. The number of contained ovals and the precise configuration of such ovals may vary according to the designed intent of the inventive haptic.

Figures 24A-B depict both top and sagittal views of a full circular haptic with arced grooves of material removed so as to provide for focal flexibility and fluid flow. In this case the number of grooves and the length and configuration of such grooves may vary in accordance with the intended purpose of the designed haptic.

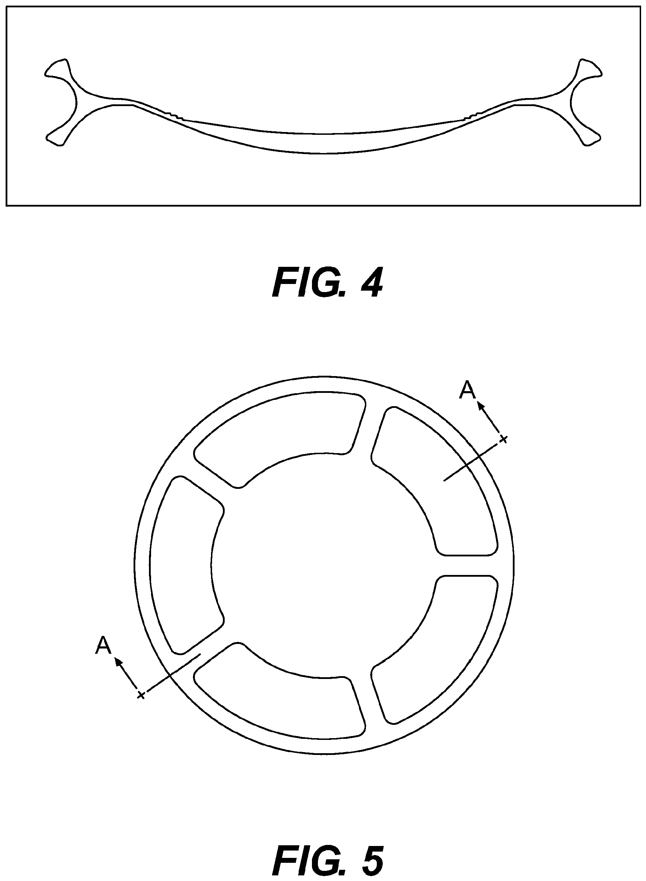

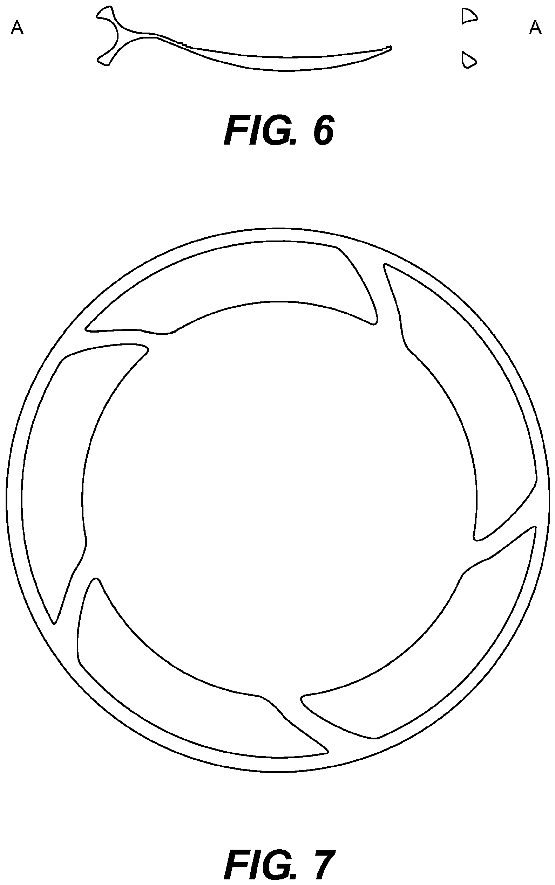

Figures 3 and 4 illustrate an emobidment of the lens, both shown in cross section. Figure 5 depicts a top view of the embodiment of the lens showing full circle anterior and posterior rings. Haptic pillars connect the rings to the haptic arms and preferably only at the haptic arms. Preferably the aperture to the fornix is significant and hydration occurs through the capsule. Also preferably, the anterior and/or posterior rings have modestly sharper edges at the contact points with the capsule. Preferably the lens is positioned close to the nucleus of the position of the natural lens and the center optic rests against the posterior capsule. The optic of this embodiment is preferably about 6 mm in diameter. Overall, this optic may provide significant improvement to depth of field vision. Figure 6 illustrates a transverse cross section from point A to A as shown in Figure 5.

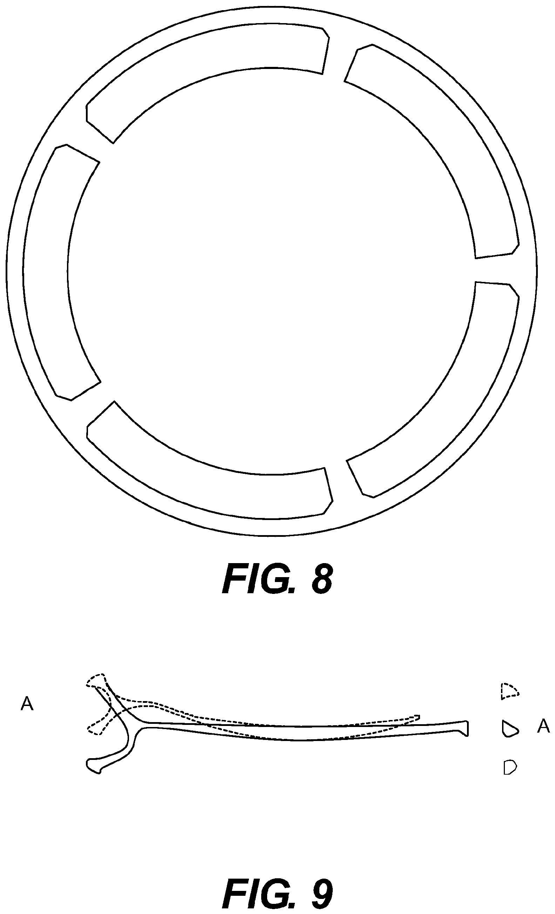

Figures 7-9 illustrate additional embodiments of inventive lenses. The lens depicted in Figure 7 has spiral anterior and posterior haptics. The haptic bridges supporting the anterior and posterior rings are angled so as to provide for some posterior compression in the event of a smaller than average capsular circumference. Figures 8 and 9 depict a lens without the angled haptic bridge supports. Figure 9 is a cross-section of the lens of figure 8. Preferably the lens has a thick anterior haptic to buttress the anterior ring, a flat anterior surface to minimize step height, and a thin bendable posterior haptic. While two shapes of haptic bridge supports are shown other haptic bridge support shapes can be used.

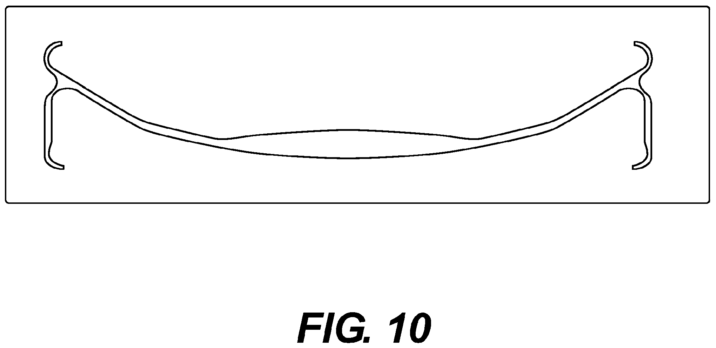

Figure 10 illustrate an inventive lens shown in cross section whose uppermost haptic is comprised of the anterior ring designed to come into contact with the anterior capsule surface, thereby arresting lens epithelial cells' migration along the anterior capsule to the fornix. This anterior ring is designed with an inventive curvature such that the ring continually maintains contact with the anterior capsule in both a distance and near vision state and in all intermediate states. The lowermost portion of the inventive haptic is comprised of the posterior ring designed to maintain contact with the posterior capsule at a point distally outward of the optical zone, thereby preventing incursion of posterior capsule opacification into the optical zone of the posterior capsule. The haptic pillar connecting the anterior and posterior rings may be solid or may have apertures cut into it, in the case of the former design, to restrict any posterior capsule opacification to the area of the fornix, or capsular equator, and in the case of the latter to permit hydration of the entire lens capsule by allowing circulation of the aqueous humor throughout the capsule. The inventive lens haptic suspends the optic posteriorly from a haptic that is connected to the haptic pillar and is placed posterior to the anterior haptic ring so as to prevent the lens optic from coming into contact with the anterior capsule. The haptic supporting the optic may be perforated in different patterns and at different intervals so as to allow circulation of the aqueous humor as well as permit the optic to move anteriorly and posteriorly within the capsule in response to the natural movements of the ciliary body so as to provide focal accommodation.

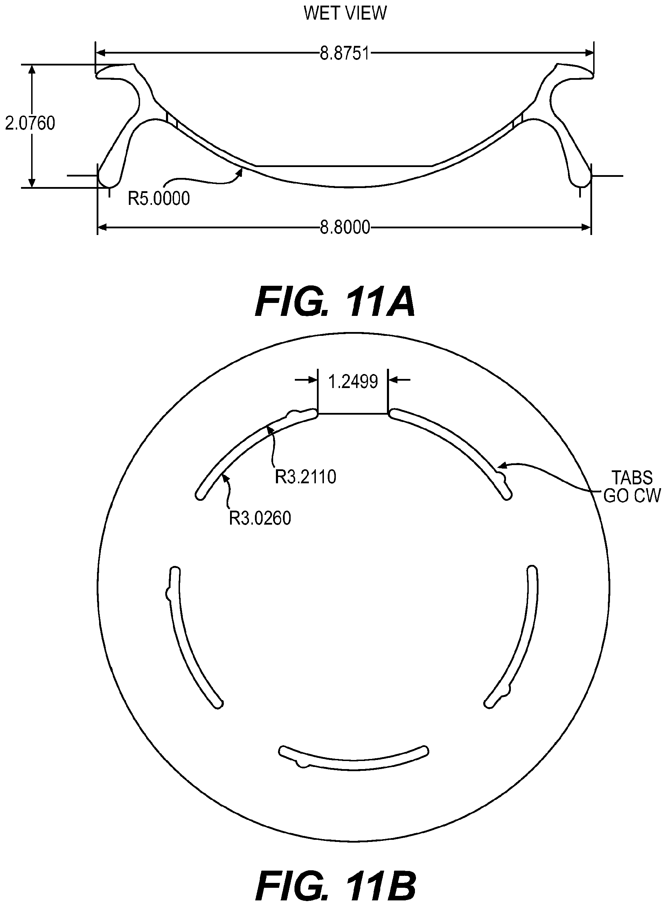

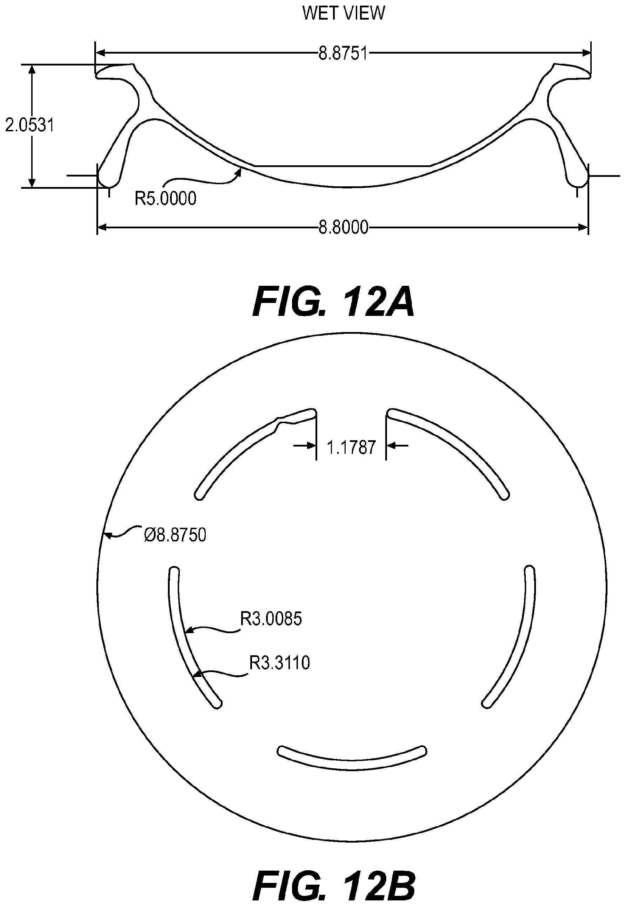

Figures 11A-B and 12A-B depict embodiments of an IOL having orientation tabs. While any IOL disclosed herein can have orientation tabs, figures 11A-B and 12A-B are examples of the IOL in figure 1 with orientation tabs. Preferably, as shown in figure 11B, the IOL has a plurality of orientation tabs. For example, as shown in figure 11B, each opening between the lens and the haptic ring may have an orientation tab positioned therein. However, as shown in figure 12B, there may be only one orientation tab. Preferably, when positioned in the eye, the orientation tabs will indicate a proper anterior and posterior position. For example, as shown in the figures, when properly implanted, the orientation tabs will be toward the clockwise end of the opening. The orientation tabs may project into the opening or extend from the opening into the IOL, or combinations thereof.

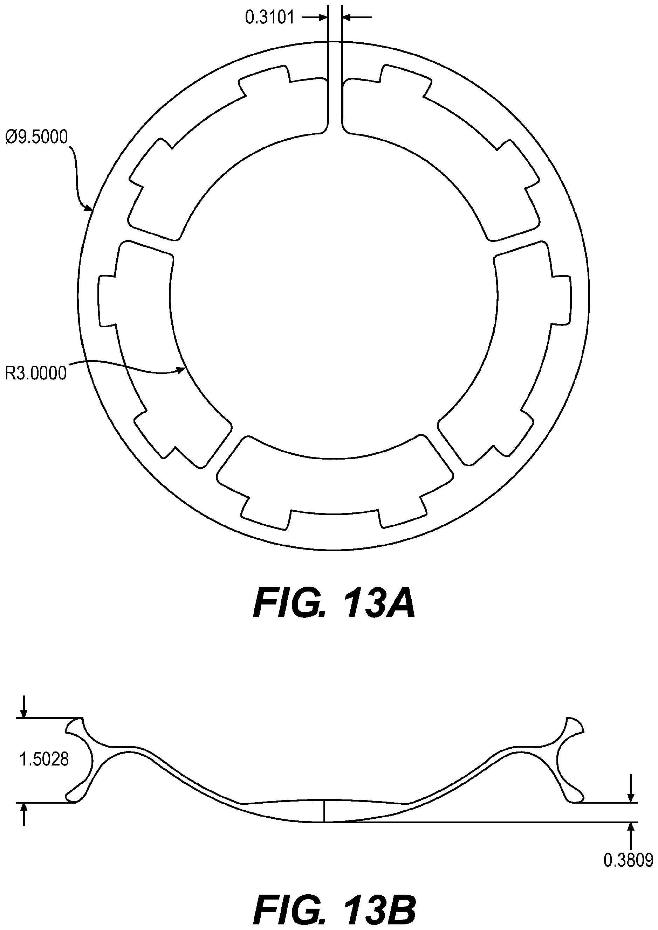

Figures 13A-B depict embodiments of an IOL having fenestrations. While any IOL disclosed herein can have fenestrations, figures 13A-B are examples of the IOL in figure 1 with orientation tabs. Preferably, as shown in figure 11A, the IOL has a plurality of fenestrations. For example, as shown in figure 11A, each opening between the lens and the haptic ring may have a pair of fenestrations positioned to extend into the haptic ring. Each opening may have more or fewer fenestrations. Preferably, the fenestrations reduce the amount of material used in the IOL and thereby reduce the weight of the IOL. Preferably, the fenestrations extend out to the fornix of the IOL.

The invention is set out in the appended claims.

## Claims

1. An intraocular lens, comprising:
an optic;
a plurality of haptic arms extending from the optic;
an annular ring coupled to the plurality of arms, wherein the annular ring is comprised of an anterior retention ring and a posterior retention ring between which an optic is suspended, wherein the anterior retention ring and the posterior retention ring are separated by a plurality of haptic pillars, wherein the plurality of haptic arms define openings between the annular ring and the optic;
and at least one orientation tab coupled to the annular ring, wherein each opening between the optic and the annular ring has an orientation tab positioned therein, wherein the orientation tabs indicate a proper anterior and posterior position, and indicate the proper alignment of the intraocular lens when placed in an eye.

2. The intraocular lens of claim 1, wherein each haptic pillar is coupled to one haptic arm.

3. The intraocular lens of claim 1, further comprising gaps between the haptic pillars.

4. The intraocular lens of claim 1, which is comprised of a hydrophilic acrylic, hydrophobic acrylic, silicone, or combinations thereof.

5. The intraocular lens of claim 1, which is comprised of a material for insertion into a human eye.

6. The intraocular lens of claim 1, further comprising a haptic ring coupling the optic to the haptic arms; optionally, wherein the haptic ring has one or more rounded corners.

7. The intraocular lens of claim 1, wherein each orientation tab extends into the annular ring.

8. The intraocular lens of claim 1, wherein each haptic arm extends from a circumference of the option to an inner surface of the annular ring.

9. The intraocular lens of claim 1, wherein the optic and the annular ring are coaxial.

10. The intraocular lens of claim 1; optionally, wherein the openings have a smaller radial width than circumferential length.

11. The intraocular lens of claim 1, wherein the annular ring is comprised of an anterior haptic ring and a posterior haptic ring connected at an apex of a "U" shaped recess in an outer circumferential surface of the annular ring; optionally, wherein the posterior haptic ring is coupled posteriorly to the anterior haptic ring.

12. The intraocular lens of claim 1, wherein the annular ring has a kidney shaped cross section.

13. The intraocular lens of claims 1, which is adapted to be compressed by an instrument to allow insertion into the eye.

14. The intraocular lens of claim 1, wherein the annular ring, when inserted into the eye, is adapted to move in response to movement of a ciliary process; optionally, wherein movement of the annular ring provides focal accommodation.

## Patentansprüche

1. Intraokularlinse, umfassend:
Eine Optik;
Eine Vielzahl haptischen Armen, die sich von der Optik erstrecken;
einen ringförmigen Ring, der an die Vielzahl von Armen gekoppelt ist, wobei der ringförmige Ring aus einem vorderen Haltering und einem hinteren Haltering besteht, zwischen denen eine Optik aufgehängt ist, wobei der vordere Haltering und der hintere Haltering durch eine Vielzahl von haptischen Säulen getrennt sind, wobei die Vielzahl von haptischen Armen Öffnung zwischen dem ringförmigen Ring und der Optik definiert;
und zumindest einen Orientierungsvorsprung, der an den ringförmigen Ring gekoppelt ist, wobei jede Öffnung zwischen der Optik und dem ringförmigen Ring einen Orientierungsvorsprung darin positioniert hat, wobei die Orientierungsvorsprünge eine geeignete vordere und hintere Position anzeigen, und die geeignete Ausrichtung der Intraokularlinse, wenn in ein Auge platziert, anzeigen.

2. Intraokularlinse nach Anspruch 1, wobei jede haptische Säule an einen haptischen Arm gekoppelt ist.

3. Intraokularlinse nach Anspruch 1, die ferner Lücken zwischen den haptischen Säulen umfasst.

4. Intraokularlinse nach Anspruch 1, die aus einem hydrophilen Acryl, hydrophoben Acryl, Silikon oder Kombinationen davon besteht.

5. Intraokularlinse nach Anspruch 1, die aus einem Material zum Einsatz in ein menschliches Auge besteht.

6. Intraokularlinse nach Anspruch 1, die ferner einen haptischen Ring umfasst, der die Optik an die haptischen Arme koppelt; optional, wobei der haptische Ring eine oder mehrere gerundete Ecken aufweist.

7. Intraokularlinse nach Anspruch 1, wobei sich jeder Orientierungsvorsprung in den ringförmigen Ring erstreckt.

8. Intraokularlinse nach Anspruch 1, wobei sich jeder haptische Arm von einem Umfang der Option zu einer Innenfläche des ringförmigen Rings erstreckt.

9. Intraokularlinse nach Anspruch 1, wobei die Optik und der ringförmige Ring koaxial sind.

10. Intraokularlinse nach Anspruch 1; optional, wobei die Öffnungen eine kleinere Radialbreite als die Umfanglänge aufweisen.

11. Intraokularlinse nach Anspruch 1, wobei der ringförmige Ring aus einem vorderen haptischen Ring und einem hinteren haptischen Ring besteht, gekoppelt an einen Scheitelpunkt einer "U"-förmigen Vertiefung in einer äußeren Umfangsfläche des ringförmigen Rings; optional, wobei der hintere haptische Ring nach hinten an den vorderen haptischen Ring gekoppelt ist.

12. Intraokularlinse nach Anspruch 1, wobei der ringförmige Ring einen nierenförmigen Querschnitt aufweist.

13. Intraokularlinse nach Anspruch 1, die angepasst ist, durch ein Instrument komprimiert zu werden, um Einsatz in das Auge zu erlauben.

14. Intraokularlinse nach Anspruch 1, wobei der ringförmige Ring, wenn in das Auge eingesetzt, angepasst ist, sich als Reaktion auf Bewegung eines Ziliarprozesses zu bewegen; optional, wobei Bewegung des ringförmigen Rings fokale Akkommodation bereitstellt.

## Revendications

1. Lentille intraoculaire, comprenant :
une optique ;
une pluralité de bras haptiques s'étendant de l'optique ;
une bague annulaire couplée à la pluralité de bras, dans laquelle la bague annulaire est composée d'une bague de retenue antérieure et d'une bague de retenue postérieure entre lesquelles une optique est suspendue, dans laquelle la bague de retenue antérieure et la bague de retenue postérieure sont séparées par une pluralité de montants haptiques, dans laquelle la pluralité de bras haptiques définissent des ouvertures entre la bague annulaire et l'optique ; et
une ou plusieurs languettes d'orientation couplées à la bague annulaire, dans laquelle chaque ouverture entre l'optique et la bague annulaire a une languette d'orientation positionnée dans celle-ci, dans laquelle les languettes d'orientation indiquent une position antérieure et postérieure correcte, et indiquent l'alignement correct de la lentille intraoculaire quand elle est placée dans un œil.

2. Lentille intraoculaire selon la revendication 1, dans laquelle chaque montant haptique est couplé à un des bras haptiques.

3. Lentille intraoculaire selon la revendication 1, comprenant en outre des espaces entre les montants haptiques.

4. Lentille intraoculaire selon la revendication 1, qui est composée d'un acrylique hydrophile, un acrylique hydrophobe, un silicone, ou des combinaisons de ces derniers.

5. Lentille intraoculaire selon la revendication 1, qui est composée d'un matériau destiné à être introduit dans un œil humain.

6. Lentille intraoculaire selon la revendication 1, comprenant en outre une bague haptique couplant l'optique aux bras haptiques ; en option, dans laquelle la bague haptique a un ou plusieurs angles arrondis.

7. Lentille intraoculaire selon la revendication 1, dans laquelle chaque languette d'orientation s'étend dans la bague annulaire.

8. Lentille intraoculaire selon la revendication 1, dans laquelle chaque bras haptique s'étend d'une circonférence de l'option à une surface intérieure de la bague annulaire.

9. Lentille intraoculaire selon la revendication 1, dans laquelle l'optique et la bague annulaire sont coaxiales.

10. Lentille intraoculaire selon la revendication 1, en option, dans laquelle les ouvertures ont une largeur radiale plus petite que la longueur circonférentielle.

11. Lentille intraoculaire selon la revendication 1, dans laquelle la bague annulaire est composée d'une bague haptique antérieure et d'une bague haptique postérieure reliées à un sommet d'un renfoncement en forme de "U" dans une surface circonférentielle extérieure de la bague annulaire ; en option, dans laquelle la bague haptique postérieure est couplée postérieurement à la bague haptique antérieure.

12. Lentille intraoculaire selon la revendication 1, dans laquelle la bague annulaire a une section transversale en forme de haricot.

13. Lentille intraoculaire selon la revendication 1, laquelle est adaptée pour être comprimée par un instrument pour permettre l'insertion dans l'œil.

14. Lentille intraoculaire selon la revendication 1, dans laquelle la bague annulaire, une fois introduite dans l'œil, est adaptée pour se déplacer en réponse au mouvement d'un processus ciliaire ; en option, dans laquelle le mouvement de la bague annulaire assure l'accommodation focale.
